# EUROPÄISCHE PATENTANMELDUNG

(11) **EP 3 542 765 A1**
(43) Veröffentlichungstag der Anmeldung: **25.09.2019**
(21) Anmeldenummer: 19155658.8
(22) Anmeldetag: 06.02.2019
(51) Int. Cl.: A61F 9/00

(54) **VORRICHTUNG ZUR INTRAVITREALEN INJEKTION IN EIN AUGE**

(30) Priorität: 23.03.2018 DE 102018107024
(71) Anmelder: Vitreoject UG (haftungsbeschränkt), 22085 Hamburg (DE)
(72) Erfinder: Kromer, Robert, 22085 Hamburg (DE)
(74) Vertreter: Uexküll & Stolberg

(57) **Zusammenfassung**

Eine Vorrichtung (2) zur intravitrealen Injektion in ein Auge, umfasst ein Gehäuse (7), das sich entlang einer Längsachse (8) erstreckt und ein proximales Ende (9) sowie ein distales Ende (10) aufweist. Am proximalen Ende (9) des Gehäuses (7) ist eine Aufnahme (11) für einen Spritzenkörper (4) mit einem darin enthaltenen Medikament angeordnet. Am distalen Ende (10) des Gehäuses (7) ist eine umlaufende Anlagefläche (12) zur Anlage der Vorrichtung (2) an einen Bereich der Bindehaut des Auges vorgesehen. Das Gehäuse (7) weist ein Reservoir (13) zur Aufnahme eines Fluids auf.

Das Gehäuse (7) umfasst weiter eine Unterdruckkammer (14), die zum distalen Ende (10) des Gehäuses (7) hin derart geöffnet ist, dass ein in der Unterdruckkammer (14) erzeugter Unterdruck die Anlagefläche (12) des Gehäuses (7) an das Auge ansaugt.

Das Reservoir (13) ist mit der Unterdruckkammer (14) derart verbunden, dass ein in der Unterdruckkammer (14) erzeugter Unterdruck Fluid aus dem Reservoir (13) in Richtung des Auges fördert und innerhalb der Anlagefläche (12) verteilt.

## Beschreibung

### Technisches Gebiet

Die vorliegende Erfindung betrifft eine Vorrichtung zur intravitrealen Injektion in ein Auge umfassend, ein Gehäuse mit einem proximalen Ende und einem distalen Ende. Am proximalen Ende des Gehäuses ist eine Aufnahme für einen Spritzenkörper mit einem darin enthaltenen Medikament angeordnet. Am distalen Ende des Gehäuses ist eine umlaufende Anlagefläche zur Anlage der Vorrichtung an einen Bereich der Bindehaut des Auges vorgesehen. Das Gehäuse weist ein Reservoir zur Aufnahme eines Fluids auf.

### Hintergrund der Erfindung

Bei einer intravitrealen Medikamentenabgabe spritzt ein Augenarzt unter örtlicher Betäubung ein Medikament in das Innere des Augapfels. Dadurch gelangt der Wirkstoff des Medikaments direkt an die Netzhaut, wodurch Nebenwirkungen in den übrigen Organen vermieden werden. Besondere chemische Zusammensetzungen der Hüllsubstanzen für einige der Wirkstoffe ermöglichen darüber hinaus eine langsame und kontinuierliche Freisetzung des Wirkstoffs. So entfaltet sich die Wirkung nach und nach und hält über längere Zeiträume an.

Mithilfe der intravitrealen Injektion werden beispielsweise altersabhängige Makuladegenerationen und Netzhautschäden, die Folge einer langjährigen Zuckerkrankheit sind, behandelt. Eine dritte Indikation sind Venenverschlüsse, die zu einer Schwellung in der Netzhautmitte (Makulaödem) führen. Schließlich können Blutungen unter der Netzhaut, die diese abzuheben und zu schädigen drohen, mithilfe einer intravitrealen Injektion behandelt werden.

Um Infektionen des Auges, beispielsweise durch Bakterien von der Augenoberfläche, zu vermeiden, wird üblicherweise vor Beginn der Injektion eine Oberflächensterilisation durchgeführt. Bei einer solchen Sterilisation werden unter anderem jodhaltige pharmazeutische Erzeugnisse eingesetzt, um das Auge sowie den daran angrenzenden Bereich wie beispielsweise die Augenlieder, die Wimpern und die Augenbrauen zu desinfizieren. Diese Sterilisationsmethode erfordert zusätzliche Verbrauchsmaterialien sowie einen erhöhten Zeitaufwand. Die damit verbundene längere Operationszeit kann das Risiko von Komplikationen erhöhen.

Eine Vorrichtung, die diesen Nachteil vermeidet, ist aus der EP 2 578 191 A1 bekannt. Die Medikamentenabgabevorrichtung ermöglicht eine lokale Desinfektion des Auges im Bereich der Einstichstelle einer Injektionsnadel. Die Medikamentenabgabevorrichtung umfasst einen Körper zur Aufnahme einer ein Medikament enthaltenen Patrone und ein mit dem Körper gekoppeltes Reservoir. Das Reservoir enthält ein Fluid, beispielsweise ein Desinfektionsmittel, und einen Kolben. Die Patrone ist mit einer Injektionsnadel verbunden und relativ zu dem Körper zwischen einer zurückgezogenen Position und einer ausgefahrenen Position axial bewegbar. Nach Aufsetzen der Vorrichtung auf die Bindehaut des Auges wird die Patrone axial von der zurückgezogenen Position in die ausgefahrende Position bewegt. Dadurch wird der Kolben ebenfalls axial verschoben und das Desinfektionsmittel aus dem Reservoir in Richtung des Auges gefördert. Das Desinfektionsmittel bedeckt den Einstichbereich der Injektionsnadel und desinfiziert diesen bevor die Injektionsnadel in das Auge eindringt.

Die Medikamentenabgabevorrichtung ist während der Injektion lediglich auf die Bindehaut aufgesetzt. Ein Verrutschen der Medikamentenabgabevorrichtung relativ zur Bindehaut ist möglich, weshalb eine ausreichende Sterilisation der Injektionsstelle nicht gewährleistet werden kann.

Es ist daher Aufgabe der vorliegenden Erfindung, eine Vorrichtung zur intravitrealen Injektion in ein Auge bereitzustellen, die ein Verrutschen relativ zum Auge verhindert und damit eine verbesserte Sterilisation ermöglicht.

### Beschreibung der Erfindung

Diese Aufgabe wird gelöst durch eine Vorrichtung mit den Merkmalen gemäß Patentanspruch 1. Bevorzugte Ausführungsformen sind in den abhängigen Patentansprüchen beschrieben.

Gemäß der vorliegenden Erfindung umfasst das Gehäuse eine Unterdruckkammer, die zum distalen Ende des Gehäuses hin derart geöffnet ist, dass ein in der Unterdruckkammer erzeugter Unterdruck die Anlagefläche des Gehäuses an das Auge ansaugt. Dadurch wird die Vorrichtung am Auge während der intravitrealen Injektion fixiert und ein Verrutschen der Vorrichtung relativ zur Bindehaut des Auges vermieden.

Erfindungsgemäß ist das Reservoir mit der Unterdruckkammer derart verbunden, dass ein in der Unterdruckkammer erzeugter Unterdruck Fluid aus dem Reservoir in Richtung des Körpers fördert und innerhalb der Anlagefläche verteilt. Bevorzugt weist das Fluid desinfizierende und/oder anästhesierende Eigenschaften auf. Beispielsweise wird als Fluid PVD-Jod verwendet. Das Anlegen eines Unterdrucks in der Unterdruckkammer bewirkt ein Austreten des Fluids aus dem Reservoir und damit eine Benetzung der Bindehaut mit dem Fluid innerhalb der Anlagefläche. Beispielsweise kann die Unterdruckkammer eine distale Öffnung aufweisen, die von der Anlagefläche umgeben ist und durch die das Fluid aus der Unterdruckkammer in Richtung des Auges austritt. Indem das Fluid mittels Unterdruck in Richtung der Bindehaut des Auges gefördert wird, wird die Sterilisation der Bindehaut im Bereich innerhalb der Anlagefläche automatisiert und dadurch vereinfacht. Eine Kontamination des Auges während der Injektion wird vermieden.

Die Aufnahme des Gehäuses ist derart ausgebildet, dass ein Spritzenkörper alleine oder zusammen mit einer Injektionsnadel in das Gehäuse eingeführt werden kann.

Die Vorrichtung kann als Einwegvorrichtung ausgebildet sein oder sich zur mehrmaligen Verwendung eignen. Soll die Vorrichtung mehrmals verbraucht werden, ist sie derart ausgebildet, dass sie nach jedem Gebrauch sterilisieret und das Fluid im Reservoir nachgefüllt werden kann.

Bevorzugt ist das Gehäuse an seinem proximalen und/oder an seinem distalen Ende vor dem ersten Gebrauch mit einem Verschluss, beispielsweise mit einer Folie, verschlossen. Der Verschluss kann beispielsweise manuell vor dem ersten Gebrauch der Vorrichtung geöffnet werden.

Im Rahmen der vorliegenden Erfindung wird unter dem Begriff "Unterdruck" ein Druck verstanden, der unter dem Umgebungsdruck von 1 bar liegt. Entsprechend wird im Rahmen der vorliegenden Erfindung unter dem Begriff "Unterdruckkammer" eine Kammer verstanden, in der der Umgebungsdruck von 1 bar reduziert werden kann und in einen Bereich zwischen 0 bar und 1 bar liegt.

Im Rahmen der vorliegenden Erfindung wird weiter unter dem Begriff "Spritzenkörper" ein hohlzylindrisch ausgebildeter Körper verstanden, an den eine Injektionsnadel (Kanüle) angesetzt und mit diesem verbunden werden kann. In einem Hohlraum des Spritzenkörpers ist ein Spritzenkolben verschiebbar angeordnet, der mittels eines rückseitig betätigbaren Stößels verschiebbar ist. Der Hohlraum kann beispielsweise ein Medikament in flüssiger Form aufnehmen, das durch Betätigung des Stößels aus dem Hohlraum und damit aus dem Spritzenkörper hinausbefördert und mithilfe der Injektionsnadel in einen Körper injiziert werden kann.

In einer Ausführungsform der vorliegenden Erfindung weist die Unterdruckkammer einen flexibel, manuell verformbaren Wandbereich auf, wobei die Unterdruckkammer derart ausgebildet ist, dass durch Verformung des Wandbereichs das Volumen der Unterdruckkammer, ausgehend von einer ursprünglichen Größe, verkleinerbar ist und durch Rückverformung des Wandbereichs das Volumen der Unterdruckkammer die ursprüngliche Größe wieder annimmt. Bevorzugt weist der Randbereich eine von einer Längsachse des Gehäuses radial nach außen konvex geformte Wandung auf. Beispielsweise wird der flexible Wandbereich von einem Arzt mit einer Hand umfasst und flexible verformt, so dass das Volumen der Unterdruckkammer relativ zu einer ursprünglichen Größe verkleinert wird. Bevorzugt wird die dabei aus der Unterdruckkammer entweichende Luft über eine innerhalb der Anlagefläche vorgesehene distale Öffnung der Unterdruckkammer bzw. des Gehäuses aus diesem hinaus befördert. Nach Ansetzen der Anlagefläche auf der Bindehaut des Auges löst der Arzt seine Umgreifung des flexiblen Wandbereichs, so dass sich dieser zumindest teilweise flexible rückverformt, um seine ursprüngliche Größe wieder anzunehmen, und dabei einen Unterdruck in der Unterdruckkammer zu erzeugen. Bevorzugt ist das Gehäuse und insbesondere der flexibel, manuell verformbare Wandbereich ergonomisch geformt. Dadurch wird eine sichere Handhabung der Vorrichtung ermöglicht.

In einer weiteren Ausführungsform der vorliegenden Erfindung ist in dem Gehäuse eine Injektionsnadel angeordnet, die sich von der Aufnahme in Richtung des distalen Endes des Gehäuses hin erstreckt, wobei die Injektionsnadel ein Verbindungsstück zum Verbinden mit dem Spritzenkörper aufweist, das in der Aufnahme des Gehäuses positioniert ist. Die Spritzenkörper weisen eine genormte Schnittstelle zur Injektionsnadel auf, so dass das in der Aufnahme befindliche Verbindungsstück der Injektionsnadel in der Lage ist, unterschiedlichste Spritzenkörper mit unterschiedlichsten Volumina aufzunehmen. Durch die Anordnung der Injektionsnadel in dem Gehäuse ist gewährleistet, dass die Injektionsnadel bis zum Zeitpunkt der Injektion in das Auge steril ist. Bevorzugt befindet sich die Injektionsnadel in einem zwischen der Aufnahme und der Anlagefläche angeordneten luftdicht verschlossenen sterilen Raum, so dass sie vor äußeren Einflüssen geschützt ist.

Bevorzugt ist eine Ausführungsform gemäß der die Aufnahme für den Spritzenkörper in dem Gehäuse entlang der Längsachse des Gehäuses von einer eingefahrenen Position in eine ausgefahrene Position verschiebbar geführt ist, wobei die Injektionsnadel in der eingefahrenen Position der Aufnahme von dem Auge beabstandet ist und in der ausgefahrenen Position der Aufnahme in das Auge eingedrungen ist. Bevorzugt weist das Gehäuse eine Begrenzung des Fahrwegs von der eingefahrenen in die ausgefahrene Position der Aufnahme auf. Beispielsweise ist diese Begrenzung einstellbar, so dass die Eindringtiefe der Injektionsnadel in das Auge vordefiniert und einstellbar ist. Durch eine geführte Verfahrbewegung der Aufnahme entlang der Längsachse des Gehäuses ist ein zielsicheres Eindringen der Injektionsnadel in das Auge gewährleistet. Ein unsachgemäßes Eindringen der Injektionsnadel in das Auge, das heißt beispielsweise ein zu tiefes Eindringen oder ein schräges Eindringen, wird somit vermieden.

Bevorzugt ist im Gehäuse ein Verfahrmechanismus zum automatischen Verfahren der Aufnahme von der eingefahrenen Position in die ausgefahrende Position angeordnet. Dadurch wird das Eindringen der Injektionsnadel in das Auge vereinfacht. Mithilfe des Verfahrmechanismus kann bevorzugt die Verfahrgeschwindigkeit der Aufnahme und/oder die Eindringtiefe der Injektionsnadel in das Auge definiert werden.

Ebenfalls bevorzugt ist eine Ausführungsform gemäß der im Gehäuse ein Rückstellmechanismus zum automatischen Verfahren der Aufnahme von der ausgefahrenen Position in die eingefahrene Position angeordnet ist. Dadurch wird ein sicheres und schnelles Herausziehen der Injektionsnadel aus dem Auge gewährleistet.

In einer weiteren Ausführungsform weist die Unterdruckkammer an einer entlang der Längsachse der Anlagefläche gegenüberliegenden Seite eine Durchgangsöffnung auf, die mit einer durchstechbaren Folie luftdicht verschlossen ist. Bevorzugt begrenzt die Durchgangsöffnung den sterilen Raum in distaler Richtung entlang der Längsachse des Gehäuses. Mithilfe der Folie wird verhindert, dass die sterile Injektionsnadel kontaminiert wird. Bevorzugt durchsticht die Injektionsnadel die Folie beim erstmaligen Gebrauch der Vorrichtung während des Verfahrens der Aufnahme von der eingefahrenen Position in die ausgefahrene Position. Darüber hinaus dient die Folie dazu, die Unterdruckkammer auf der der Anlagefläche gegenüberliegenden Seite derart zu verschließen, dass ein Unterdruck in der Unterdruckkammer erzeugt werden kann.

Ebenfalls bevorzugt ist eine Ausführungsform bei der das Reservoir an die Unterdruckkammer angrenzend, in einer Wandung des Gehäuses angeordnet ist. Bevorzugt ist das Reservoir derart ausgebildet, dass es 0,1 bis 3 ml von Fluid aufnehmen kann. Die an die Unterdruckkammer angrenzende Positionierung des Reservoirs ist platzsparend und ermöglicht ein Benetzen der Bindehaut des Auges mit dem Fluid ohne vorher lange Wege zurücklegen zu müssen.

In einer weiteren Ausführungsform weist das Gehäuse an dem distalen Ende eine Positionierungseinrichtung zur Positionierung der Vorrichtung auf der Bindehaut des Auges auf. Dadurch wird erreicht, dass der Arzt die Vorrichtung leicht und gezielt an der richtigen Position auf der Bindehaut des Auges positionieren kann. Dies gewährleistet eine sichere intravitreale Injektion in ausreichender Entfernung von relevanten intraokularen Strukturen.

Bevorzugt ist die Positionierungseinrichtung als Außenkontur des Gehäuses am distalen Ende des Gehäuses ausgebildet, wobei die Außenkontur derart geformt ist, dass sie zumindest teilweise eine Außenkontur einer Iris und/oder zumindest teilweise einen Verlauf eines Übergangs zwischen Augenlidern und Bindehaut eines Auges nachempfunden ist. Dies ermöglicht eine einfache Positionierung der Vorrichtung auf der Bindehaut des Auges. Ist die Außenkontur des distalen Endes des Gehäuses zumindest teilweise einer Außenkontur einer Iris nachempfunden, so muss der Arzt zur richtigen Positionierung der Vorrichtung auf der Bindehaut des Auges lediglich die Außenkontur des Gehäuses mit der Außenkontur der Iris überlagern. Ist die Außenkontur am distalen Ende des Gehäuses zumindest teilweise ein Verlauf eines Übergangs zwischen Augenlidern und Bindehaut des Auges nachempfunden so muss der Arzt vor der intravitrealen Injektion lediglich das distale Ende des Gehäuses an den Übergang zwischen den Augenlidern und der Bindehaut des Auges anlegen, um die richtige Positionierung auf der Bindehaut des Auges zu finden. Dadurch ist eine deutlich vereinfachte und schnellere Positionierung der Vorrichtung auf der Bindehaut des Auges gewährleistet.

Die Erfindung wird nachfolgend anhand von in den Figuren dargestellten bevorzugten Ausführungsformen erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstands dar. Es zeigen:
- Fig. 1: eine Schnittansicht einer erfindungsgemäßen Vorrichtung mit einer Spritze,
- Fig. 2: eine schematische Darstellung eines Auges und einem an das Auge angesetzten distalen Ende des Gehäuses in einer Schnittansicht,
- Fig. 3: eine schematische Darstellung eines Auges und eines an das Auge angelegten distalen Endes eines Gehäuses gemäß einer zweiten Ausführungsform in einer Schnittansicht,
- Fig. 4: eine Schnittansicht einer zweiten Ausführungsform der erfindungsgemäßen Vorrichtung,
- Fig. 5: eine Schnittansicht einer dritten Ausführungsform der erfindungsgemäßen Vorrichtung,
- Fig. 6: eine Schnittansicht einer vierten Ausführungsform der erfindungsgemäßen Vorrichtung und
- Fig. 7: eine Schnittansicht einer fünften Ausführungsform der erfindungsgemäßen Vorrichtung.

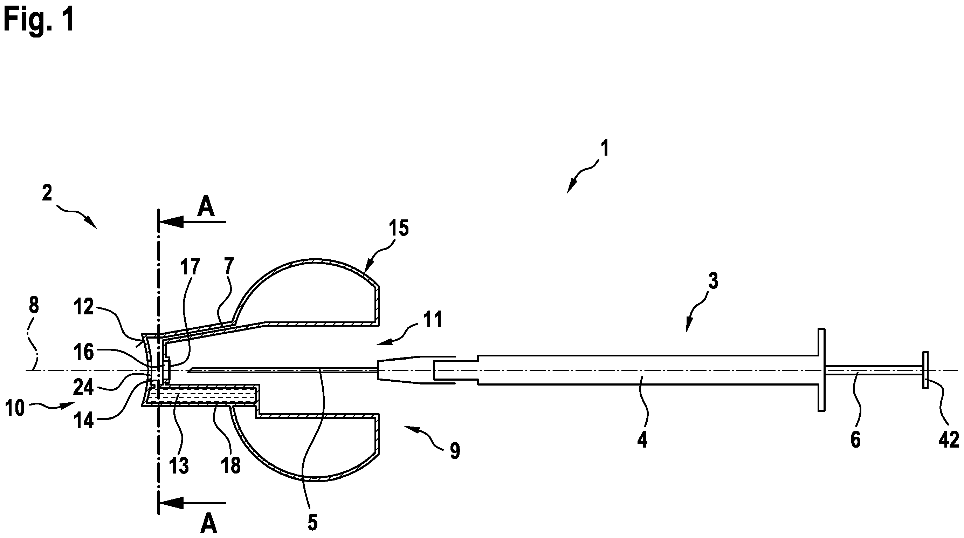

Fig. 1 zeigt ein Medikamentenabgabesystem 1 umfassend eine Vorrichtung 2 zur intravitrealen Injektion in ein Auge (nicht dargestellt) und eine Spritze 3 mit einem Spritzenkörper 4 und einer damit verbundenen Injektionsnadel 5. Der Spritzenkörper 4 weist einen Kolben 6 und einen Stößel 42 auf, mittels derer ein in dem Spritzenkörper 4 enthaltenes Medikament durch die Injektionsnadel 5 in das Auge injiziert werden kann.

Die Vorrichtung 2 umfasst ein Gehäuse 7, das sich entlang einer Längsachse 8 erstreckt. Das Gehäuse weist ein proximales Ende 9 und ein distales Ende 10 auf. Am proximalen Ende 9 des Gehäuses 7 ist eine Aufnahme 11 für den Spritzenkörper 4 mit dem darin enthaltenen Medikament und der mit dem Spritzenkörper 4 verbundenen Injektionsnadel 5 angeordnet. Am distalen Ende 10 des Gehäuses 7 ist eine umlaufende Anlagefläche 12 zur Anlage der Vorrichtung 2 an einen Bereich der Bindehaut des Auges (nicht dargestellt) vorgesehen. Die Anlagefläche 12 kann kreisförmig oder oval geformt sein oder andere geometrische Formen aufweisen.

Das Gehäuse 7 weist weiter ein Reservoir 13 zur Aufnahme eines Fluids auf. Bei dem Fluid kann es sich beispielsweise um ein oberflächendesinfizierendes Erzeugnis zur bereichsweisen Desinfizierung der Bindehaut des Auges, insbesondere PVD-Jod, und/oder ein oberflächenanästhesierendes Erzeugnis zur zumindest teilweisen Anästhesie der Bindehaut des Auges handeln.

Das Gehäuse 7 umfasst weiter eine Unterdruckkammer 14, die über eine Öffnung 24 zum distalen Ende 10 des Gehäuses 7 hin derart geöffnet ist, dass ein in der Unterdruckkammer 14 erzeugte Unterdruck die Anlagefläche 12 des Gehäuses 7 an die Bindehaut des Auges ansaugt. Die Öffnung 24 ist quer zur Längsachse 8 des Gehäuses 7 von der Anlagefläche 12 umgeben.

Die Unterdruckkammer 14 weist einen flexibel, manuell verformbaren Wandbereich 15 auf. Die Unterdruckkammer 14 ist derart ausgebildet, dass durch Verformung des Wandbereichs 15 das Volumen der Unterdruckkammer 14, ausgehend von einer ursprünglichen Größe, verkleinerbar ist und durch Rückverformung des Wandbereichs 15 das Volumen der Unterdruckkammer 14 die ursprüngliche Größe wieder annimmt.

Die Unterdruckkammer 14 hat an einer der Anlagefläche 12 gegenüberliegenden Seite eine Durchgangsöffnung 16, die mit einer mittels der Injektionsnadel 5 durchstechbaren Folie 17 luftdicht verschlossen ist.

Das Reservoir 13 ist an die Unterdruckkammer 14 angrenzend, in einer Wandung 18 des Gehäuses 7 angeordnet. Das Reservoir 13 ist mit der Unterdruckkammer 14 derart verbunden, dass ein in der Unterdruckkammer 14 erzeugter Unterdruck Fluid aus dem Reservoir 13 in Richtung des Auges fördert und innerhalb der Anlagefläche 12, d.h. im Bereich der Öffnung 24 der Unterdruckkammer 14, verteilt. Beispielsweise kann das Reservoir 13 mit der Unterdruckkammer 14 über ein sich bei einem anliegenden Unterdruck öffnendes Verbindungselement verbunden sein. Ein solches Verbindungselement kann beispielsweise ein sich bei Unterdruck öffnendes Ventil sein.

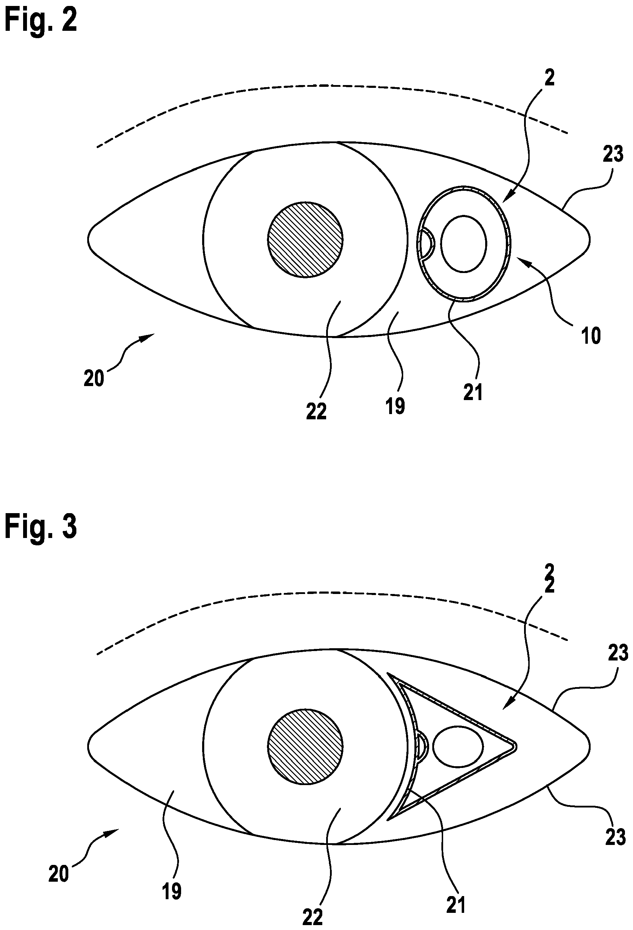

Figur 2 zeigt eine schematische Darstellung eines Auges 20 mit einer Bindehaut 19 und einem auf die Bindehaut 19 aufgesetzten distalen Ende 10 des Gehäuses 7 der Vorrichtung 2 im Schnitt entlang der Linie A-A aus Figur 1.

Die Vorrichtung 2 weist an ihrem distalen Ende 10 eine als Außenkontur 21 ausgebildete Positionierungseinrichtung zur Positionierung der Vorrichtung 2 auf der Bindehaut 19 des Auges 20 auf. Die Außenkontur 21 besitzt eine ovale Form und wird bei richtiger Positionierung der Vorrichtung 2 derart auf der Bindehaut 19 positioniert, dass sie mittig zwischen der Iris 22 und den beiden Augenlidern 23 angeordnet ist. Die Öffnung 24 der Unterdruckkammer 14 (Figur 1) ist oval.

Figur 3 zeigt eine zweite Ausführungsform der Außenkontur 21 am distalen Ende 10 der Vorrichtung 2. Die Außenkontur 21' unterscheidet sich von der in Figur 2 gezeigten dadurch, dass sie zumindest teilweise einer Außenkontur der Iris 22 und zumindest teilweise einem Verlauf eines Übergangs zwischen den Augenlidern 23 und der Bindehaut 19 des Auges 20 nachempfunden ist. Die Außenkontur 21' weist die Form eines gleichschenkligen Dreiecks mit einer gebogenen Grundseite auf.

Im Folgenden wird die Funktionsweise der Vorrichtung unter Bezugnahme auf die Figuren 1 bis 3 erläutert:
Der flexible Wandbereich 15 der Unterdruckkammer 14 wird von einem Arzt einhändig umgriffen und manuell derart verformt, so dass das Volumen der Unterdruckkammer 14 verringert wird und die in der Unterdruckkammer 14 befindliche Luft durch die von der Anlagefläche 12 umgebenen Öffnung 24 zumindest teilweise entweicht. Die Anlagefläche 12 wird dann auf die Bindehaut 19 eines Auges 20 aufgesetzt und anhand ihrer Außenkontur 21, 21' auf der Bindehaut 19 positioniert. Löst der Arzt seinen Griff um den flexiblen Wandbereich 15 der Vorrichtung 2, verformt sich dieser zumindest annähernd in seine Ausgangsform zurück. Da die Anlagefläche 12 luftdicht auf der Bindehaut 19 aufliegt, kann jedoch keine oder kaum Luft in die Unterdruckkammer 14 einströmen, so dass in der Unterdruckkammer 14 ein Unterdruck entsteht. Die Anlagefläche 12 saugt sich und damit die Vorrichtung 2 an der Bindehaut 19 fest. Zeitgleich bewirkt der Unterdruck, dass Fluid in Form von einem desinfizierenden und/oder anästhesierendem Erzeugnis in Richtung des Auges 20, insbesondere in die von der Anlagefläche 12 umgebenden Öffnung 24 gefördert und dadurch die Bindehaut 19 mit dem Fluid benetzt wird. Das Fluid desinfiziert bzw. anästhesiert die Bindehaut 19 im Bereich der Öffnung 24.

Die Spitze 3 wird nun in die Aufnahme 11 des Gehäuses 7 derart eingeführt, das die Injektionsnadel 5 die Folie 17 durchsticht und die Injektionsnadel 5 durch die Durchgangsöffnung 16 sowie durch die von der Anlagefläche 12 umgebenen Öffnung 24 in die Bindehaut 19 des Auges 20 eindringt. Ist die gewünschte Eindringtiefe der Injektionsnadel 5 erreicht, wird der Stößel 42 und damit der Kolben 6 relativ zum Spritzenkörper 4 betätigt, so dass das im Spritzenkörper 4 befindliche Medikament über die Injektionsnadel 5 in das Auge 20 injiziert wird.

Nach erfolgter Injektion des Medikaments wird die Spritze 3 manuell aus dem Auge 20 herausgezogen. Da der Innendruck im Auge 20 höher ist als der Umgebungsdruck, wird die Eintrittswunde der Injektionsnadel 5 sofort nach deren Herausziehen aus dem Auge 20 verschlossen. Zum Lösen der Vorrichtung 2 von der Bindehaut 19 des Auges 20 wird der flexibel verformbare Wandbereich 15 der Unterdruckkammer 14 erneut händisch verformt, so dass der Druck in der Unterdruckkammer 14 ansteigt und das Niveau des Umgebungsdrucks erreicht. Die Vorrichtung 2 kann dann von der Bindehaut 19 abgehoben werden.

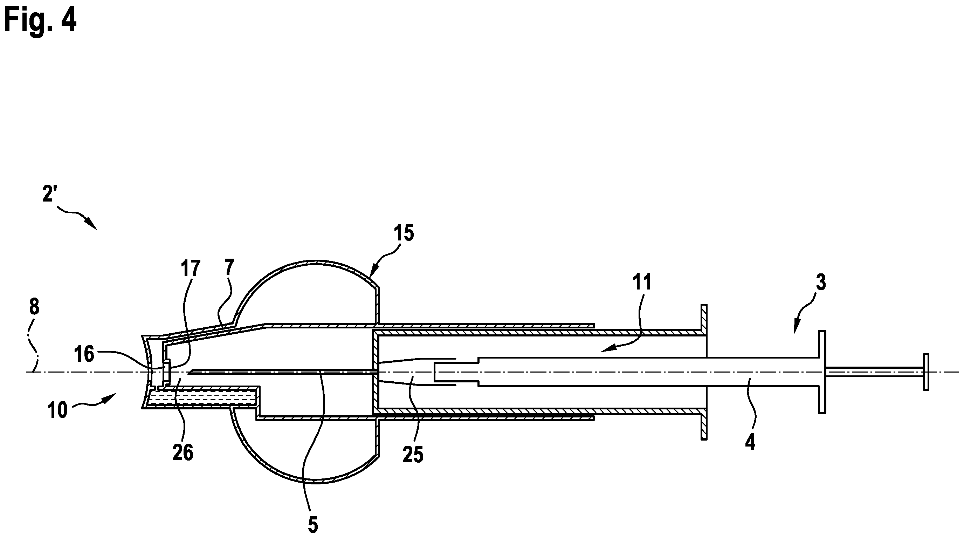

Figur 4 zeigt eine zweite Ausführungsform der Vorrichtung 2. Die Vorrichtung 2' unterscheidet sich von der in Figur 1 gezeigten dadurch, dass in dem Gehäuse 7 eine Injektionsnadel 5 angeordnet ist, die sich von der Aufnahme 11 in Richtung des distalen Endes 10 des Gehäuses 7 hin erstreckt. Die Injektionsnadel 5 weist ein Verbindungstück 25 zum Verbinden mit dem Spritzenkörper 4 auf, das in der Aufnahme 11 positioniert ist.

Ein weiterer Unterschied besteht darin, dass die Aufnahme 11 für den Spritzenkörper 4 in dem Gehäuse 7 entlang der Längsachse 8 des Gehäuses 7 von einer eingefahrenen Position in eine ausgefahrene Position verschiebbar geführt ist. In der eingefahrenen Position ist die Injektionsnadel 5 von dem Auge 20 (Figur 2) beabstandet. In der ausgefahrenen Position ist die Injektionsnadel 5 in das Auge 20 eingedrungen. Optional ist die Aufnahme 11 zylindrisch ausgebildet und umgibt den Spritzenkörper 4 zumindest teilweise, wobei das Verbindungsstück 25 in der Aufnahme 11 angeordnet ist.

Ein weiterer Unterschied im Vergleich zur Vorrichtung 2 besteht darin, dass die Injektionsnadel 5 in einem sterilen Raum 26 im Inneren des Gehäuses 7 angeordnet ist. Der Raum 26 erstreckt sich im Gehäuse 7 von der Aufnahme 11 entlang der Längsachse 8 des Gehäuses 7 bis hin zu der Durchgangsöffnung 16, die mit der Folie 17 verschlossen ist.

Die Funktion der Vorrichtung 2' unterscheidet sich von der in Figur 1 gezeigten Vorrichtung 2 dadurch, dass der Spritzenkörper 4 wahlweise vor bzw. nach Fixierung der Vorrichtung 2' auf der Bindehaut 19 des Auges 20 mit der im Gehäuse 7 befindlichen Injektionsnadel 5 über das Verbindungsstück 25 verbunden wird. Durch manuelle Kraftausübung auf die Aufnahme 11 und/oder den Spritzenkörper 4 entlang der Längsachse 8 und in Richtung des Auges 20 verfährt die Aufnahme 11 entlang der Längsachse 8 relativ zu dem Gehäuse 7 von der eingefahrenen Position in die ausgefahrene Position, durchsticht die Folie 17 und dringt in das Auge 20 (Figur 2) ein. Die Eindringtiefe kann optional, beispielsweise durch einen Anschlag für die Aufnahme 11 im Gehäuse 7, begrenzt werden. Nach erfolgter intravitrealer Injektion des Medikaments wird durch erneute Kraftausübung entlang der Längsachse 8 in eine Richtung vom Auge 20 weg die Aufnahme 11 von der ausgefahrenen Position in die eingefahrene Position verfahren und die Injektionsnadel 5 aus dem Auge 20 herausgezogen.

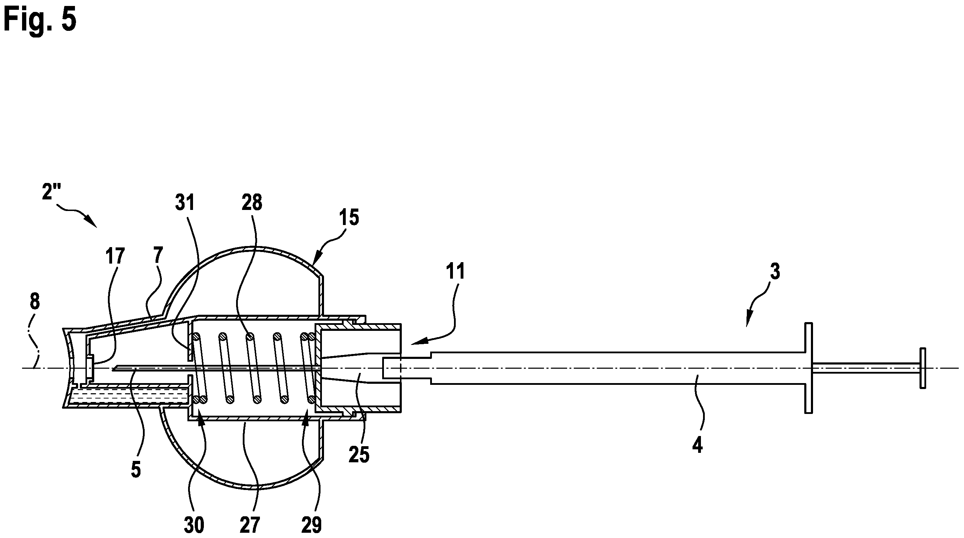

Figur 5 zeigt eine dritte Ausführungsform der erfindungsgemäßen Vorrichtung 2 gemäß Figur 1. Die Vorrichtung 2" unterscheidet sich von der in Figur 4 gezeigten Vorrichtung 2' dadurch, dass in dem Gehäuse 7 ein Rückstellmechanismus 27 zum automatischen Verfahren der Aufnahme 11 von der ausgefahrenen Position in die eingefahrene Position angeordnet ist. Der Rückstellmechanismus 27 weist eine Feder 28 auf, die an einem ersten Ende 29 an der Aufnahme 11 sowie an einem dem ersten Ende 29 gegenüberliegenden zweiten Ende 30 der Feder 28 an Vorsprüngen 31 des Gehäuses 7 gelagert ist.

Die Funktion der Vorrichtung 2" unterscheidet sich von der in Figur 3 gezeigten Vorrichtung 2' dadurch, dass während des Verfahrens der Aufnahme 11 von der eingefahrenen Position in die ausgefahrene Position die Feder 27 komprimiert wird und dadurch eine Rückstellkraft in Richtung der eingefahrenen Position der Aufnahme 11 auf diese ausübt. Nach der intravitrealer Injektion des Medikaments und nachdem keine händische Kraft mehr auf die Aufnahme 11 ausgeübt wird, bewirkt die Rückstellkraft der Feder 27 ein automatisches Verfahren der Aufnahme 11 von der ausgefahrenen Position zurück in die eingefahrene Position und damit ein automatisches Herausziehen der Injektionsnadel 5 aus dem Auge 20.

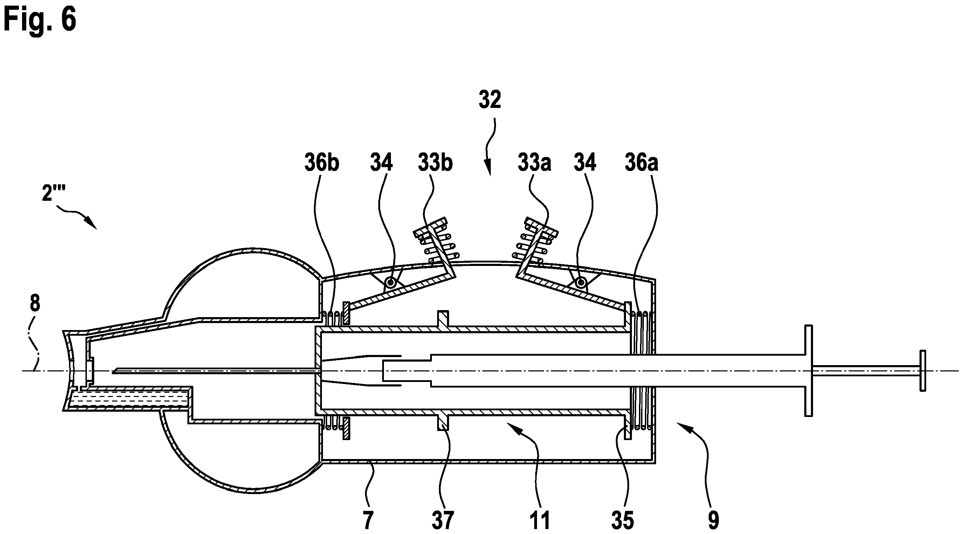

Figur 6 zeigt eine vierte Ausführungsform der erfindungsgemäßen Vorrichtung 2 gemäß Figur 1. Die Vorrichtung 2"' unterscheidet sich von der in Figur 5 gezeigten Vorrichtung 2" dadurch, dass im Gehäuse 7 ein Verfahrmechanismus 32 zum automatischen Verfahren der Aufnahme 11 von der eingefahrenen Position in die ausgefahrene Position angeordnet ist. Der Verfahrmechanismus 32 umfasst einen ersten Kipphebel 33, der um ein Gelenk 34 am Gehäuse 7 kippbar gelagert ist. Der erste Kipphebel 33 blockiert einen Kragen 35 der Aufnahme 11 am proximalen Ende 9 des Gehäuses 7. Zwischen dem Kragen 35 und dem proximalen Ende 9 des Gehäuses 7 ist eine erste vorgespannte Feder 36a angeordnet, die den Kragen 35 und damit die Aufnahme 11 mit einer Federkraft in Richtung des Auges 20 beaufschlagt.

Die Vorrichtung 2"' unterscheidet sich weiter von der in Figur 5 gezeigten dadurch, dass der Rückstellmechanismus 27 einen zweiten Kipphebel 33b aufweist, der ebenfalls um ein Gelenk 34 im Gehäuse 7 kippbar gelagert ist. Der Kipphebel 33b blockiert eine sich gegenüber dem Gehäuse abstützende zweite vorgespannte Feder 36b, die bei einer in der ausgefahrenen Position befindlichen Aufnahme 11 und nach Betätigung des zweiten Kipphebels 33b gegen Anschläge 37 stößt und ausgebildet ist, die Aufnahme 11 von der ausgefahrenen Position in die eingefahrene Position zu verfahren.

Die Funktionsweise der Vorrichtung 2"' unterscheidet sich von der in Figur 5 gezeigten dadurch, dass der Verfahrmechanismus 32 durch Betätigung des ersten Kipphebels 33a den Kragen 35 der Aufnahme 11 freigibt. Dadurch entspannt sich die vorgespannte erste Feder 36a und verfährt die Aufnahme 11 von der eingefahrenen Position in die ausgefahrene Position entlang der Längsachse 8 des Gehäuses 7. Daraufhin dringt die Injektionsnadel 5 durch die Folie 17 und die Öffnung 24 in die Bindehaut 19 des Auges 20 (Figur 2) ein. Der maximale Verfahrweg der Aufnahme 11 entlang der Längsachse 8 kann optional, beispielsweise durch Anschläge im Gehäuse 7 begrenzt werden.

Nach der intravitrealen Injektion des Medikaments wird durch Betätigung des zweiten Kipphebels 33b der Rückstellmechanismus 27 aktiviert. Durch Betätigung des zweiten Kipphebels 33b wird die zweite vorgespannte Feder 36b freigegeben, die gegen Anschläge 37 der Aufnahme 11 stößt und die Aufnahme 11 von der ausgefahrenen Position in die eingefahrene Position verfährt. Es versteht sich, dass die vorgespannte Feder 36b derart dimensioniert ist, dass sie die erste Feder 36a bei Verfahren der Aufnahme 11 von der ausgefahrenen Position in die eingefahrene Position komprimiert.

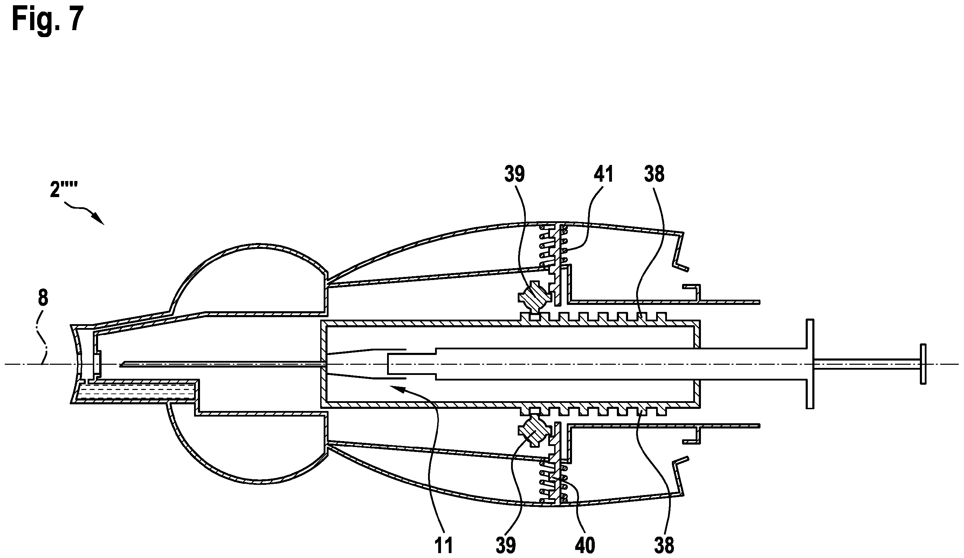

Figur 7 zeigt eine fünfte Ausführungsform der erfindungsgemäßen Vorrichtung gemäß Figur 1. Die Vorrichtung 2"" unterscheidet sich von der in Figur 6 gezeigten Vorrichtung 2"' dadurch, dass der Verfahrmechanismus 32 und der Rückstellmechanismus 27 erste Zahnstangen 38 aufweisen, die an der Aufnahme 11 befestigt sind. Der Verfahrmechanismus 32 sowie der Rückstellmechanismus 27 weisen weiter Zahnräder 39 auf, die über zweite Zahnstangen 40 betätigbar sind und mit den ersten Zahnstangen 38 in Eingriff stehen.

Die Funktionsweise der Vorrichtung 2"" unterscheidet sich von der in Figur 6 gezeigten dadurch, dass durch radiales Verschieben der zweiten Zahnstangen 40 quer zur Längsachse 8 des Gehäuses 7 die Zahnräder 39, die mit den ersten Zahnstangen 38 kämmen, in Drehung versetzt werden und dadurch die Aufnahme 11 entlang der Längsachse 8 von der eingefahrenen in die ausgefahrene Position verschoben wird. Beim radialen Verschieben der zweiten Zahnstangen 40 werden Federn 41 komprimiert, so dass nach der intravitrealen Injektion und bei Freigabe der zweiten Zahnstangen 40 die Federn 41 diese radial nach außen quer zur Längsachse 8 des Gehäuses 7 drücken, wodurch die Zahnräder 39 in Drehung versetzt werden und die Aufnahme 11 von der ausgefahrenen Position in die eingefahrene Position verfahren wird.

### Bezugszeichenliste

- 1: Medikamentenabgabesystem
- 2-2"": Vorrichtung
- 3: Spritze
- 4: Spritzenkörper
- 5: Injektionsnadel
- 6: Kolben
- 7: Gehäuse
- 8: Längsachse
- 9: proximales Ende (Gehäuse)
- 10: distales Ende (Gehäuse)
- 11: Aufnahme (Gehäuse)
- 12: Anlagefläche (Gehäuse)
- 13: Reservoir
- 14: Unterdruckkammer
- 15: Wandbereich (Unterdruckkammer)
- 16: Durchgangsöffnung
- 17: Folie
- 18: Wandung
- 19: Bindehaut
- 20: Auge
- 21, 21': Positioniereinrichtung
- 22: Iris
- 23: Augenlider
- 24: Öffnung (Unterdruckkammer)
- 25: Verbindungsstück
- 26: steriler Raum
- 27: Rückstellmechanismus
- 28: Feder
- 29: erstes Ende (Feder)
- 30: zweites Ende (Feder)
- 31: Vorsprünge
- 32: Verfahrmechanismus
- 33 a/b: erster/zweiter Kipphebel
- 34: Gelenk
- 35: Kragen
- 36 a/b: erste/zweite vorgespannte Feder
- 37: Anschläge
- 38: erste Zahnstange
- 39: Zahnräder
- 40: zweite Zahnstange
- 41: Feder
- 42: Stößel

## Patentansprüche

1. Vorrichtung zur intravitrealen Injektion in ein Auge (20), umfassend:
ein Gehäuse (7), das sich entlang einer Längsachse (8) erstreckt und ein proximales Ende (9) sowie ein distales Ende (10) aufweist,
wobei am proximalen Ende (9) des Gehäuses (7) eine Aufnahme (11) für einen Spritzenkörper (4) mit einem darin enthaltenen Medikament angeordnet ist,
wobei am distalen Ende (10) des Gehäuses (7) eine umlaufende Anlagefläche (12) zur Anlage der Vorrichtung (2, 2', 2", 2"', 2"") an einen Bereich der Bindehaut (19) des Auges (20) vorgesehen ist und,
wobei das Gehäuse (7) ein Reservoir (13) zur Aufnahme eines Fluids aufweist,
**dadurch gekennzeichnet,**
**dass** das Gehäuse (7) weiter eine Unterdruckkammer (14) umfasst, die zum distalen Ende (10) des Gehäuses (7) hin derart geöffnet ist, dass ein in der Unterdruckkammer (14) erzeugter Unterdruck die Anlagefläche (12) des Gehäuses (7) an das Auge (20) ansaugt und,
**dass** das Reservoir (13) mit der Unterdruckkammer (14) derart verbunden ist, dass ein in der Unterdruckkammer (14) erzeugter Unterdruck Fluid aus dem Reservoir (13) in Richtung des Auges (20) fördert und innerhalb der Anlagefläche (12) verteilt.

2. Vorrichtung nach Anspruch 1, **dadurch gekennzeichnet, dass** die Unterdruckkammer (14) einen flexibel, manuell verformbaren Wandbereich (15) aufweist, wobei die Unterdruckkammer (14) derart ausgebildet ist, dass durch Verformung des Wandbereichs (15) das Volumen der Unterdruckkammer (14), ausgehend von einer ursprünglichen Größe, verkleinerbar ist und durch Rückverformung des Wandbereichs (15) das Volumen der Unterdruckkammer (14) die ursprüngliche Größe wieder annimmt.

3. Vorrichtung nach einem der vorhergehenden Ansprüche, **dadurch gekennzeichnet, dass** in dem Gehäuse (7) eine Injektionsnadel (5) angeordnet ist, die sich von der Aufnahme (11) in Richtung des distalen Endes (10) des Gehäuses (7) hin erstreckt, wobei die Injektionsnadel (5) ein Verbindungstück (25) zum Verbinden mit dem Spritzenkörper (4) aufweist, das in der Aufnahme (11) des Gehäuses (7) positioniert ist.

4. Vorrichtung nach Anspruch 3, **dadurch gekennzeichnet, dass** die Aufnahme (11) für den Spritzenkörper (4) in dem Gehäuse (7) entlang der Längsachse (8) des Gehäuses (7) von einer eingefahrenen Position in eine ausgefahren Position verschiebbar geführt ist, wobei die Injektionsnadel (5) in der eingefahrenen Position der Aufnahme (11) von dem Auge (20) beabstandet ist und in der ausgefahrenen Position der Aufnahme (11) in das Auge (20) eingedrungen ist.

5. Vorrichtung nach Anspruch 4, **dadurch gekennzeichnet, dass** im Gehäuse (7) ein Verfahrmechanismus (32) zum automatischen Verfahren der Aufnahme (11) von der eingefahrenen Position in die ausgefahrene Position angeordnet ist.

6. Vorrichtung nach Anspruch 4, **dadurch gekennzeichnet, dass** im Gehäuse (7) ein Rückstellmechanismus (27) zum automatischen Verfahren der Aufnahme (11) von der ausgefahrenen Position in die eingefahrene Position angeordnet ist.

7. Vorrichtung nach einem der vorhergehenden Ansprüche, **dadurch gekennzeichnet, dass** die Unterdruckkammer (14) an einer der Anlagenfläche (12) gegenüberliegenden Seite eine Durchgangsöffnung (16) aufweist, die mit einer durchstechbaren Folie (17) luftdicht verschlossen ist.

8. Vorrichtung nach einem der vorhergehenden Ansprüche, **dadurch gekennzeichnet, dass** das Reservoir (13) an die Unterdruckkammer (14) angrenzend, in einer Wandung (18) des Gehäuses (7) angeordnet ist.

9. Vorrichtung nach einem der vorhergehenden Ansprüche, **dadurch gekennzeichnet, dass** das Gehäuse (7) an dem distalen Ende (10) eine Positionierungseinrichtung (21) zur Positionierung der Vorrichtung (2, 2', 2", 2"', 2"") auf der Bindehaut (19) des Auges (20) aufweist.

10. Vorrichtung nach Anspruch 9, **dadurch gekennzeichnet, dass** Positionierungseinrichtung als Außenkontur (21) am distalen Ende (10) des Gehäuses (7) ausgebildet ist, wobei die Außenkontur (21) derart geformt ist, dass sie zumindest teilweise einer Außenkontur einer Iris (22) und/oder zumindest teilweise einem Verlauf eines Übergangs zwischen Augenlidern (23) und Bindehaut (19) eines Auges (20) nachempfunden ist.
